# EUROPÄISCHE PATENTSCHRIFT

(11) **EP 1 842 505 B1**
(45) Veröffentlichungstag und Bekanntmachung des Hinweises auf die Patenterteilung: **15.04.2009**
(21) Anmeldenummer: 07007013.1
(22) Anmeldetag: 04.04.2007
(51) Int. Cl.: A61B 19/02, A61C 19/02, A61L 2/26

(54) **Verpackungsvorrichtung für Implantatschrauben**
Packaging device for implant screws
Dispositif d'emballage pour vis d'implant

(30) Priorität: 07.04.2006 DE 102006016866
(43) Veröffentlichungstag der Anmeldung: 10.10.2007
(60) Teilanmeldung: 08006951.1
(73) Patentinhaber: MEDICON EG CHIRURGIEMECHANIKER-GENOSSENSCHAFT, D-78532 Tuttlingen (DE)
(72) Erfinder: Burger, Andreas, 78532 Tuttlingen (DE); Wenzler, Klaus, 78665 Frittlingen (DE); Schröder, Kristine, 78567 Fridingen (DE); Hettich, Petra, 78665 Frittlingen (DE); Liebermann, Karl-Ernst, 78570 Mühlheim (DE)
(74) Vertreter: Mammel, Ulrike

(56) Entgegenhaltungen:
- EP-A1- 1 023 909
- WO-A-99/02196
- WO-A-20/05092231
- DE-A1- 10 230 519
- DE-A1- 19 735 147
- GB-A- 2 418 421
- US-A- 5 829 590
- US-A- 6 116 452
- US-A- 6 138 850
- US-B1- 6 783 004

## Beschreibung

Die vorliegende Erfindung betrifft eine Verpackung zur Aufnahme von Implantatschrauben.

Schraubenverpackungen können beispielsweise in der Traumatologie, der Mund-, Kiefer-, Gesichts-Chirurgie, der Hals-Nasen-Ohren-Chirurgie, der Neurochirurgie, der Schädelbasis-Chirurgie, der Handchirurgie und der okuloplastischen Chirurgie eingesetzt werden.

In der Regel sind Verpackungen für Implantatschrauben zur Aufnahme geringer Stückzahlen von Schrauben ausgelegt. Damit das Aus- und Umsortieren der Schrauben aus Plastikverpackungen vor der OP in die jeweiligen Lagerungssysteme entfallen kann, sind die Verpackungen so gestaltet, dass sie in die speziellen, während der OP eingesetzten Lagerungssysteme eingesetzt und die Schrauben in diesen Verpackungen in den Lagerungssystemen gereinigt und sterilisiert werden können.

Die Schraubenverpackung ist somit selbst Teil des sterilisierbaren Lagerungssystems.

Nachdem die Schrauben auch noch im OP in der ursprünglichen Verpackung mit der vollständigen Herstellerkennzeichnung aufbewahrt sind, sind die während einer OP verwendeten Schrauben durch ihre Chargen- oder Artikelnummer identifizierbar und rückverfolgbar, was zwischenzeitlich für eine jede bei einer Operation verwendete Schraube in Deutschland dokumentiert sein sollte.

Weiterhin sind die Verpackungen so gestaltet, dass die Schrauben während der Operation auf einfache Weise entnommen werden können. Im Allgemeinen befindet sich der Schraubenschaft einer jeden Schraube in einer entsprechenden Bohrung, und der Schraubenkopf ragt zur einfacheren Entnahme nach oben aus der Verpackung heraus.

Aus der WO 2005/092231 ist ein Behältersystem zur Aufnahme von Schrauben bekannt, das aus mehreren miteinander separierbar über Träger oder Abdeckungen verbundenen Behältern besteht. Diese separierbaren Behälter dienen dazu, eine jede einzelne Schraube jeweils in einem einzelnen Behälter darzubieten. Die Abdeckungen sind auf die Träger aufgeclipst. Mehrere Einzelträger, die jeweils eine Schraube umfassen, sind miteinander über die Träger selbst oder die Abdeckungen der jeweiligen Träger verbunden, wobei die miteinander separierbar verbundenen Einzelbehälter in einem gemeinsamen U-förmigen Sockel aufgenommen sein können. Die Schraubengewinde der einzelnen in den einzelnen Trägern aufgenommenen Schrauben werden dabei in dem gemeinsamen Hohlraum des U-förmigen Sockels aufgenommen. Die einzelnen Träger sind in dem massiven U-förmigen Sockel, der einen Standfuß aufweist, verschiebbar.

Eine Verpackung für mehrere Schrauben mit einem verschiebbaren Deckel ist aus der GB 2 418 421 bekannt.

Bereits 1993 wurden von der Firma Titamed N. V., Belgien, sterilisierbare zylindrische Kunststoffkörper mit Bohrungen in der Stirnfläche angeboten, die zur Aufnahme von Schrauben dienten.

Aus dem Gebrauchsmuster DE 297 05 944 U1 und Prospekten der Firma Gebrüder Martin GmbH & Co. KG sind Schraubenverpackung, die jeweils zur Aufnahme von fünf Schrauben dienen, bekannt. Die Verpackung besteht aus einem flachen, quaderförmigen Grundkörper, in dem schmale, den Körper vollständig durchquerende Längsbohrungen zur Aufnahme der Schrauben vorgesehen sind. Rechtwinklig zu den Längsbohrungen, die zur Aufnahme der Schraubenachse dienen, sind Querbohrungen vorgesehen, die die Schraubenachse durchqueren. Zwischen den Querbohrungen verbleiben Wandabschnitte, die einzelne Gewindeabschnitte umgeben.

Um ein Herausfallen der Schrauben während des Transports zu verhindern weisen die Schraubenverpackungen einen vollständig abnehmbaren Deckel auf.

Die Schraubenverpackungen werden in entsprechenden Öffnungen im Lochboden der Lagerungssysteme aufgenommen, wobei die Schraubenverpackungen mit seitlich vorgesehenen Laschen in Öffnungen im Boden eingeclipst werden können. Die Oberseite der Schraubenverpackung mit den nach oben weisenden Schraubenköpfen steht über den Boden der Lagerungssysteme heraus.

Die Reinigung und Sterilisierung der Implantatschrauben in der Verpackung erfolgt im Wesentlichen durch den Eintritt von Reinigungsflüssigkeit und Dampf in die Querbohrungen und die schmale Längsbohrung.

Bei diesen bekannten Schraubenverpackungen kommt es während der Operation teilweise zu einem Verschmutzen der in der Verpackung befindlichen Schrauben mit Blut, wenn an dem Schraubenzieher haftendes Blut auf den Boden zwischen den Schraubenverpackungen tropft und dann durch die Querbohrungen an die Schraubenschafte gelangt.

Nachteilig ist bei diesen Schraubenverpackungen auch, dass diese in eine Vielzahl von kleinen, rasterförmig angeordneten Öffnungen, etwa der Größe von 30 mm x 5 mm, im Lochboden des Lagerungssystems von oben eingesetzt werden müssen, was Konzentration und eine ruhige Hand erfordert.

Ein weiterer wesentlicher Nachteil dieser bekannten Schraubenverpackung sind auch die schlechte Reinig- und Sterilisierbarkeit der Schrauben. Im Allgemeinen erfolgt zunächst eine Reinigung der Schrauben in der Verpackung in dem Lagerungssystem mit einer Reinigungsflüssigkeit in einer Art Spülmaschine, und anschließend findet die Dampfsterilisation statt, wobei bei der Dampfsterilisation eine Temperatur von 134 °C über eine Zeitdauer von wenigstens 5 Minuten in der Umgebung der Schrauben erreicht werden muss.

Die Lagerungssysteme der Gebrüder Martin GmbH & Co. KG selbst weisen neben dem Boden mit Öffnungen zum Durchtritt der Reinigungsflüssigkeit und Öffnungen im Boden zur Aufnahme der Schraubenverpackungen starre vollflächige Seitenwände auf. Von oben können die Lagerungssysteme mit einem aufschieb- oder aufclipsbaren Deckel, der ebenfalls eine Vielzahl kleiner Öffnungen zum Durchtritt der Reinigungsflüssigkeit und des Dampfes aufweist, verschlossen werden.

Wahlweise können in diese Lagerungssysteme weitere Einsätze, beispielsweise für Biegeschablonen, Knochenplatten oder zur Instrumentenlagerung eingebracht werden. Auch diese Einsätze sind kastenförmig mit starren Wänden und einer Lochbodenplatte ausgestattet. Die Einsätze werden von oben in die Öffnung bzw. bei Aufteilung des Lagerungssystems zur Lagerung zweier Einsätze in eine der Öffnungen eingesetzt.

Die Aufgabe der vorliegenden Erfindung besteht darin, Schraubenverpackungen bereitzustellen, die sich durch eine einfachere Handhabbarkeit und bessere Reinig- und Sterilisierbarkeit auszeichnen.

Diese Aufgabe wird durch die Merkmale des unabhängigen Anspruchs gelöst.

Die Halteeinrichtung für die Schrauben weist im Allgemeinen Aufnahmen für mehrere Schrauben auf, die vorzugsweise entlang einer Geraden angeordnet sind. Je nach Einsatzzweck kann eine solche Halteeinrichtung Aufnahmen für im Allgemeinen etwa 3 bis 10 Schrauben umfassen, es ist jedoch ebenfalls möglich, Aufnahmen für nur zwei Schrauben oder Aufnahmen für mehr als 10 Schrauben vorzusehen. Bevorzugt sind Aufnahmen für 5 oder 10 Schrauben.

Die Aufnahmen dienen dazu, die Schrauben in ihrem oberen Bereich, d.h. im Bereich des unteren Schraubenkopfes oder im Übergangsbereich Schraubenkopf/Schaft oder im oberen Bereich des Schraubenschaftes, zu halten. Die Aufnahmen können als Bohrungen, beispielsweise in einem horizontalen Wandabschnitt der Halteeinrichtung, ausgestaltet sein, in die die Schraubenschafte eingesteckt werden, bis der untere Teil des Schraubenkopfes auf der Oberfläche des Wandabschnitts aufliegt.

Der Abstand der Bohrungen zueinander entspricht vorzugsweise annähernd einem Bohrungsdurchmesser. Infolge der vorzugsweise linearen Anordnung der Bohrungen weist die gesamte Verpackung eine langgestreckte Form auf.

Auch kann die Aufnahme bzw. Fixierung der Schrauben beispielsweise durch Aussparungen, beispielsweise Schlitze, quadratische, rechteckige, dreieckige oder ovale Durchbrüche erfolgen.

Die Aufnahmen dienen dazu, die Schrauben in ihrer Vertikalposition zu halten und ein seitliches Verrutschen zu verhindern.

Der weitaus längste Teil des Schraubenschaftes ragt somit von unten aus der Aufnahme oder Bohrung ins Freie; der Zutritt der Reinigungsflüssigkeit und des Dampfes bei der Reinigung/Sterilisation an den Schraubenschaft ist somit ungehindert.

Um während des Transports oder der Lagerung der Schrauben in der Verpackung eine Beschädigung der nach unten aus der Aufnahme herausstehenden Schraubenschafte und eine Verletzung des Bedienpersonals an den Schraubengewinden und den herausstehenden Schraubenspitzen zu verhindern, weist die Halteeinrichtung eine Schutzabdeckung auf, mit der der Bereich der Schraubenschafte abgedeckt werden kann. Diese Schutzabdeckung dient auch dazu, eine Beschädigung der die Verpackung während des Transports und der Lagerung häufig umhüllenden Plastikfolie durch hervorstehende Schraubenschafte zu verhindern.

Die Schutzabdeckung weist eine Öffnung zur Befestigung an der Halteeinrichtung auf.

Die Schutzabdeckung kann auf die Halteeinrichtung aufgesteckt, aufgeschoben, eingerastet oder auf sonstige Weise lösbar mit der Halteeinrichtung befestigt werden.

Eine bevorzugte Ausführungsform sieht vor, die Schutzabdeckung auf die Halteeinrichtung aufzuschieben, wobei das Schieben/Montieren vorzugsweise in Längsrichtung der Halteeinrichtung erfolgt. In dieser bevorzugten Ausführungsform ist die Schutzabdeckung ein Gehäuse mit einer Öffnung, wobei zusätzlich ein rechtwinklig zur Verschiebe-/Montagerichtung verlaufender Wandabschnitt freigeschnitten ist. Der freigeschnittene Wandabschnitt dient dazu, den Eintritt der Schraubenschafte in das Gehäuse beim Einschieben zu ermöglichen.

Ist die Schutzabdeckung auf die Halteeinrichtung aufgeschoben, so sind bis auf die Seite, durch die die Schafte in den Deckel eingeschoben wurden, alle die Schraubenschafte umhüllenden Flächen geschlossen.

Das seitliche Aufschieben der Schutzabdeckung hat gegenüber einem Aufstecken des Deckels "von unten" den Vorteil, dass durch die korrespondierenden Schiebeflächen an den Schutzabdeckungen/Halteeinrichtungen eine Führung beim Einschieben erfolgt, d.h. das Einschieben wird gegenüber einem Aufstecken der Schutzabdeckung von unten erheblich erleichtert. Im letzteren Fall müsste die Schutzabdeckung im freien Raum ohne Hilfsmittel, wie Führungsflächen, parallel zu den Schraubenschaftspitzen orientiert und dann aufgeschoben werden, was Konzentration und manuelle Geschicklichkeit voraussetzt.

Um eine einfache Verbindung zwischen der Halteeinrichtung und der Schutzabdeckung durch Verschieben zu ermöglichen, weist die Halteeinrichtung vorzugsweise an den horizontalen Wandabschnitt angrenzend einen vertikalen Wandabschnitt auf, der sich um die Außenkante des horizontalen Wandabschnitts umlaufend in Richtung der Schraubenschafte erstreckt. An dem vertikalen Wandabschnitt sind Führungsmittel vorgesehen, die mit korrespondierenden Führungsmitteln an der Schutzabdeckung und/oder dem nachfolgend erläuterten Deckel korrespondieren.

Die zur Aufnahme der Abdeckung vorgesehene Führungsmittel an der Halteeinrichtung können ebenfalls auch zur Aufnahme der Führungsmittel des Lagerungssystems, insbesondere deren Grundkörper, dienen. So können zur Lagerung der Schraubenverpackung in der Grundplatte des Lagerungssystems bei entfernter Schutzabdeckung die der länglichen Öffnung in der horizontalen Platte benachbarten Randbereiche in den Führungsmittel, die ansonsten zur Aufnahme der Schutzabdeckung dienen, eingreifen.

Ein weiterer Vorteil der Verbindung von Halteeinrichtung und Schutzabdeckung durch seitliches Verschieben besteht darin, dass die Schutzabdeckung selbst von einer bereits in einem Lagerungssystem eingefügten Verpackung noch nachträglich durch seitliches Verschieben entfernt werden kann, was bei einer von unten aufgesteckten oder eingerasteten Schutzabdeckung schon wegen der fehlenden Zugänglichkeit nicht möglich wäre.

Erfindungsgemäß ist an der Halteeinrichtung eine Informationsfläche vorgesehen, beispielsweise durch Verbreiterung des horizontalen Wandabschnitts oder in Form eines horizontalen Stegabschnitts an dem vertikalen Wandabschnitt, auf der die die Schrauben betreffenden Informationen wie LOT-Nummer, Chargen-Nummer sowie ggf. Schraubenlänge angebracht sein können. Die vom Hersteller bestückten und beschrifteten Verpackungen mit den jeweiligen Schrauben werden in der Verpackung für die betreffende Operation in die entsprechenden Lagerungssysteme einsortiert, wobei Schutzabdeckung und Deckel vor dem Einsortieren oder wenigstens vor der Reinigung/Sterilisation entfernt werden.

Nachdem die Schrauben weiterhin in der Halteeinrichtung, die auch die Informationsfläche umfasst, lagern, kann auch während der Operation jederzeit bestimmt werden, Schrauben welcher LOT- und Chargen-Nummer implantiert wurden, d.h. die Rückverfolgbarkeit ist auf einfache Weise möglich.

Um ein Herausfallen der Schrauben aus der Verpackung zu verhindern, sollte die Verpackung zusätzlich einen Deckel aufweisen. Dieser Deckel kann ebenfalls aufgeschoben, aufgedrückt, aufgeclipst, eingerastet oder auf sonstige Weise mit der Halteeinrichtung auf der der Schutzabdeckung gegenüberliegenden Seite der Halteeinrichtung verbunden werden.

Vorzugsweise wird auch der Deckel auf die Aufnahme seitlich aufgeschoben, wie es bereits bei der Schutzabdeckung erläutert wurde.

Prinzipiell entsprechen sich die Gestaltung der Schutzabdeckung und des Deckels, beide sind jeweils quaderförmig, weisen jeweils eine Öffnung für die Schraubenköpfe bzw. Schraubenschafte auf und einen freigeschnittenen Wandabschnitt, durch den der Durchtritt der Schraubenköpfe oder Schafte erfolgt.

Eine weitere Ausführungsform sieht vor, die Verpackungen selbst oder wenigstens Bereiche der Halteinrichtung mit einer Farbkodierung zu versehen, die einem bestimmten Schraubendurchmesser entspricht. Diese Farbkodierung kann sich auch in einer entsprechenden Farbkodierung von Einsätzen für Knochenplatten und chirurgischen Instrumenten fortsetzen, wobei einem jeden Schraubendurchmesser eine bestimmte Farbe zugeordnet wird. Hierdurch können Operationszeiten verkürzt und langes Suchen nach Implantaten, Schrauben und Instrumenten für die jeweiligen Durchmesser vermieden und das Risiko der Implantation falscher Schrauben ausgeschlossen werden.

Eine weitere Variante sieht vor, Messskalen zur Bestimmung der Schraubenlänge an den Verpackungsvorrichtungen vorzusehen.

Durch die Möglichkeit, Lagerungssysteme mit verschiedenen Verpackungen, die die gewünschten Schrauben in der gewünschten Länge, dem gewünschten Durchmesser und der gewünschten Anzahl umfassen, auf einfache Weise zu bestücken, können vor jeder OP mit geringem Zeitaufwand individuell die voraussichtlich benötigten Schrauben in dem Lagerungssystem zusammengestellt und die Schrauben dann sorgfältig gereinigt und sterilisiert werden, wobei die Identifizierbarkeit der Schrauben während der OP erhalten bleibt.

Wenn die erfindungsgemäße Verpackung auch im Wesentlichen in Bezug auf Schrauben erläutert wurde, so eignen sich die in Lagerungssystemen aufnehmbaren Verpackungen auch für andere implantierbare Verbindungsmittel wie beispielsweise Nägel, Stifte oder Klammern, wobei die Aufnahme für die Köpfe der Nägel oder Stifte oder die Halterung für die Klammern selbstverständlich an deren Gestaltung anzupassen ist.

Die Schraubenverpackungen können in einem neuartigen Lagerungssystem der hiesigen Anmelderin gelagert werden, das nicht - wie aus dem Stand der Technik hinlänglich bekannt - massive Seitenwände aufweist, sondern das vielmehr durch wenigstens vier senkrecht zur Bodenplatte verlaufende stegförmige Hauptabschnitte gekennzeichnet ist, die als Führungsflächen für die innerhalb des Halteabschnitts angeordneten Einlegeböden, Einlegetische, Tabletts, Unterteilungen etc. dienen können, wobei durch eine große Öffnung oder einen großen Freischnitt zwei Hauptabschnitten ein sehr großer Durchtritt der Reinigungsflüssigkeit in der Spülmaschine und des Dampfes gewährleistet ist. Die Aufnahme der zuvor beschriebenen Schraubenverpackungen kann in einem tischartigen Einsatz erfolgen Der Tisch kann vier Beine und eine rechteckige oder quadratische horizontale Platte aufweisen. An zwei einander gegen-überliegenden Seiten dieser Platte erstrecken sich mehrere längliche Öffnungen, die dazu dienen, durch Einschieben die zuvor beschriebenen Schraubenverpackungen aufzunehmen.

Dadurch, dass das Lagerungssystem keine Seitenwände aufweist, ist die seitliche Zugänglichkeit zu den Schraubenverpackungen und auch ein Wiederbefüllen durch Einschieben einer neuen Verpackung in das Lagerungssystem selbst mit darin angeordnetem tischartigen Einsatz möglich. Hierzu muss nur ein die Öffnungsmulden nach außen abschließender Schwenkarm ausgeschwenkt werden, so dass die Schraubenverpackungen aus den betreffenden länglichen Öffnungen herausgeschoben werden können.

Prinzipiell ist es ebenfalls möglich, die erfindungsgemäßen Schraubenverpackungen von oben in entsprechende Öffnungen in einen Einsatz einzustecken. Allerdings wird bei solch einer Ausführungsform auf die Führung beim Einschieben und die freie Zugänglichkeit von der Seite verzichtet.

Anstelle des zuvor beschriebenen tischartigen Einsatzes besteht prinzipiell auch die Möglichkeit, eine tablettartige Vorrichtung mit Öffnungen zur Aufnahme der Schraubenverpackungen seitlich, wie bereits beim Deckel beschrieben, in entsprechende Führungsnuten in das Lagerungssystem einzuschieben. Auf die zuvor beschriebenen Füße kann bei solch einem Schiebeeinsatz verzichtet werden.

Ein Tisch mit Füßen hat jedoch gegenüber dieser Einschiebelösung den Vorteil, dass er als solcher mit darin befindlicher Schraubenverpackung an einem anderen Ort aufgestellt werden kann, was bei einem einschiebbaren Tablett mit Öffnungen zur Aufnahme der Schraubenverpackungen nicht möglich ist.

Die Erfindung wird nachfolgend anhand von Ausführungsbeispielen näher beschrieben.

Es zeigen:
- **Fig. 1:**: eine Seitenansicht der Schraubenverpackung im Schnitt mit Halteeinrichtung, Schraube, Schutzabdeckung und Deckel,
- **Fig. 2:**: eine Draufsicht auf die Halteeinrichtung mit zehn Bohrungen von oben,
- **Fig. 3:**: eine perspektivische Ansicht der Schutzabdeckung,
- **Fig. 4:**: eine perspektivische Seitenansicht der Schraubenverpackung mit Schrauben, teilweise eingeschobenem Deckel und teilweise eingeschobener Schutzabdeckung,
- **Fig. 5:**: eine perspektivische Ansicht des Grundkörpers des Lagerungssystems,
- **Fig. 6:**: eine Seitenansicht des Grundkörpers aus Fig. 5 mit aufgeschobenem Deckel,
- **Fig. 7:**: eine perspektivische Ansicht des Lagerungssystems mit Grundkörper, Deckel und Einsatz zur Lagerung der erfindungsgemäßen Schraubenverpackung,
- **Fig. 8:**: einen Einsatz in den Grundkörper zur Lagerung der erfindungsgemäßen Schraubenverpackung aus Fig. 1 bis 4 und
- **Fig. 9:**: eine Draufsicht auf den Einsatz zur Lagerung der erfindungsgemäßen Schraubenverpackung (mit eingeschobenen Schraubenverpackungen).

Die Vorrichtung 11 zum Verpacken, Aufbewahren und/oder Lagern von Implantat-Schrauben, -Stiften und Klammern (nachfolgend "Schraubenverpackung") umfasst eine Halteeinrichtung 12, eine Schutzabdeckung 13 und einen Deckel 22.

Die Halteeinrichtung 12 weist einen horizontalen Wandabschnitt 19 mit mehreren Bohrungen 16 auf, die dazu dienen, die in die Bohrungen 16 einsteckbaren Schrauben 14 zu halten. Hierzu ist der Durchmesser der Bohrungen 16 geringfügig größer als der Durchmesser 35 des Schraubenschaftes am Übergang zum Schraubenkopf 23, so dass die Schraube 14 mit der Unterseite des Schraubenkopfes 23 auf der nach oben weisenden Fläche des horizontalen Wandabschnitts 19 aufsitzt. Nach unten ragen Abschnitte 15 der Schrauben 14, insbesondere ein Großteil des Schraubenschaftes, aus der Halteeinrichtung 12 heraus.

Entlang der gesamten nach außen weisenden Kante des horizontalen Wandabschnitts 19 verläuft ein starr mit dem Wandabschnitt 19 verbundener vertikaler Wandabschnitt 20. Die Halteeinrichtung 12 ist somit annähernd quaderförmig und weist eine nach unten offene Fläche auf.

An dem vertikalen Wandabschnitten 20 sind Führungsmittel 24, 25, 28, 29 vorgesehen, die dazu dienen, mit komplementären Führungsmitteln 30, 31 an der Schutzabdeckung 13 entsprechend einer Nut-Feder-Verbindung zusammenzuwirken. Auch sind an den vertikalen Wandabschnitten 20 Führungsmittel 25, 26, 27, 28 vorgesehen, die dazu dienen, mit komplementären Führungsmittel 32, 33 an dem Deckel 22 zu korrespondieren.

Die Dicke des vertikalen Wandabschnitts 20 und des horizontalen Wandabschnitts 19 beträgt vorzugsweise etwa 1 mm.

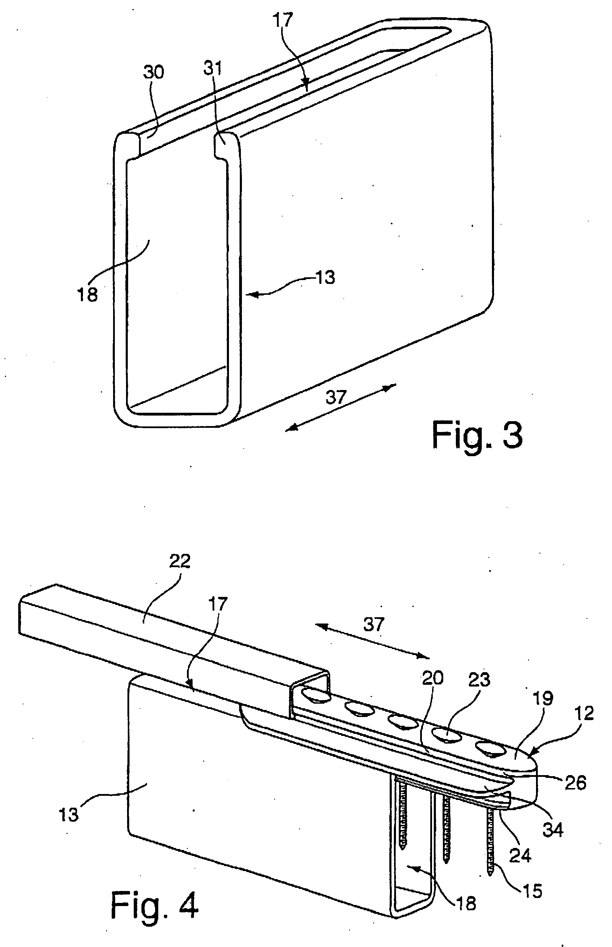

Die Schutzabdeckung 13 und der Deckel 22 werden vorzugsweise in Montage-/Verschieberichtung 37 in Fig. 4 auf die Halteeinrichtung 12 seitlich aufgeschoben.

Die Schutzabdeckung 13 und der Deckel 22, die schematisch in Figur 3 dargestellt sind, sind ein Gehäuse mit einer Öffnung 17, die dazu dient, mit der Halteeinrichtung 12 verbunden zu werden. Zusätzlich ist ein rechtwinklig zur Schiebe-Montageeinrichtung 37 verlaufender Wandabschnitt 18 freigeschnitten, der bei den erfindungsgemäßen Schiebemechanismen für Deckel und Schutzabdeckung das Einschieben der herausstehenden Schraubenköpfe und -schäfte ermöglicht.

Das eine Führungsmittel 25 geht in einen Stegabschnitt 34 über, der dazu dient, an seiner Oberseite mit den jeweiligen Chargen-/LOT-Nummern beschriftet zu werden.

Dadurch, dass keine direkte Verbindung zwischen dem vertikalen Wandabschnitt 20 und den herabhängenden Schraubenschäften 15 besteht, wird verhindert, dass während der Operation vom Schraubenzieher herabtropfendes Blut an die Schraubenschafte gelangen kann. Zudem wird ein Herunterlaufen von Blut auf einer Seite der Vorrichtung auch noch durch den Stegabschnitt 34 verhindert.

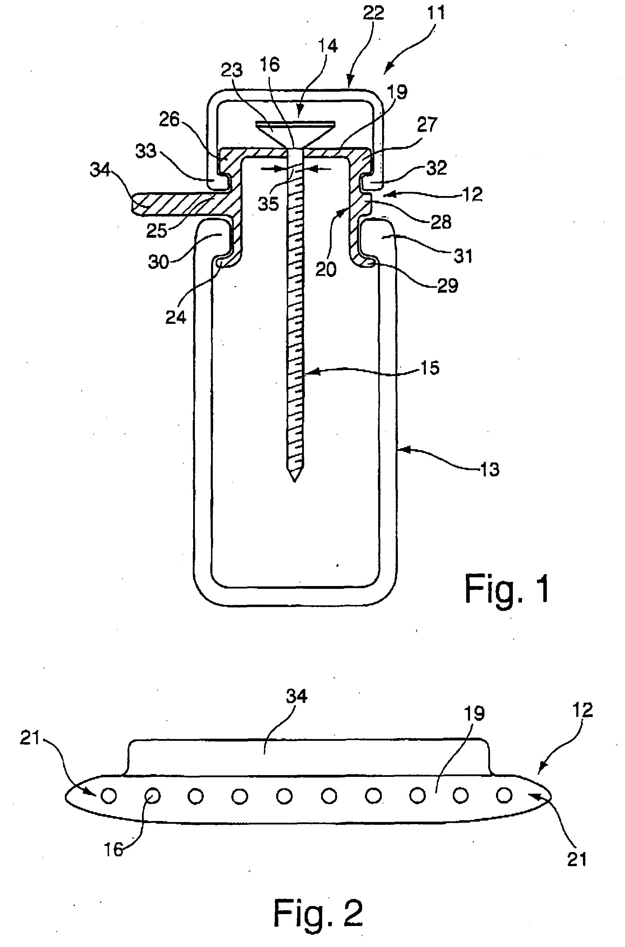

Soll eine Schraubenverpackung in der horizontalen Platte 82 des Lagerungssystems gelagert werden, so wird die Schraubenverpackung mit Schrauben 14, ggf. mit Deckel 22, aber ohne Schutzabdeckung 13 seitlich in eine längliche Öffnung 83 in der horizontalen Platte 82 eingeschoben, so dass die Randbereiche der Platte 82 entlang der länglichen Öffnung 83 entsprechend der Führungsmittel 30, 31 in der Schutzabdeckung 13 in Figur 1 in der Nut zwischen den Führungsmitteln 25, 24 und 28, 29 aufgenommen werden.

Die nach unten weisende Seite des Stegabschnitts 34 kann weiterhin dann, wenn die Vorrichtung in einem entsprechenden Lagerungssystem gelagert ist, als Auflagefläche dienen.

Ebenfalls zur Auflage in einem entsprechenden Lagerungssystem können die nach unten weisenden Flächen der beiden Außenflächen 21 in dem horizontalen Wandabschnitt 19 dienen.

Die Größe der Vorrichtung ist von der Anzahl der darin aufzubewahrenden Schrauben und deren Durchmesser abhängig.

Bei einer Anzahl von 10 Schrauben und einem Durchmesser der Bohrungen 16 für die Schrauben von ca. 2,5 mm beträgt die Gesamtlänge der Vorrichtung ca. 55 mm, die Höhe ca. 30 mm und die Tiefe ca. 8 mm.

Bei dieser Ausführungsform entspricht der Abstand der Bohrungen 16 zueinander in etwa dem Durchmesser der Bohrung.

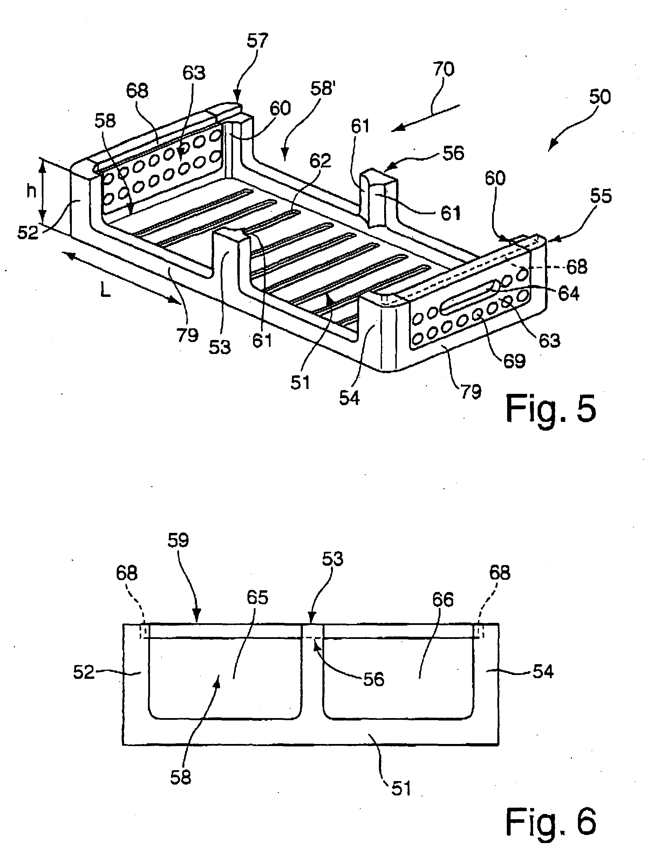

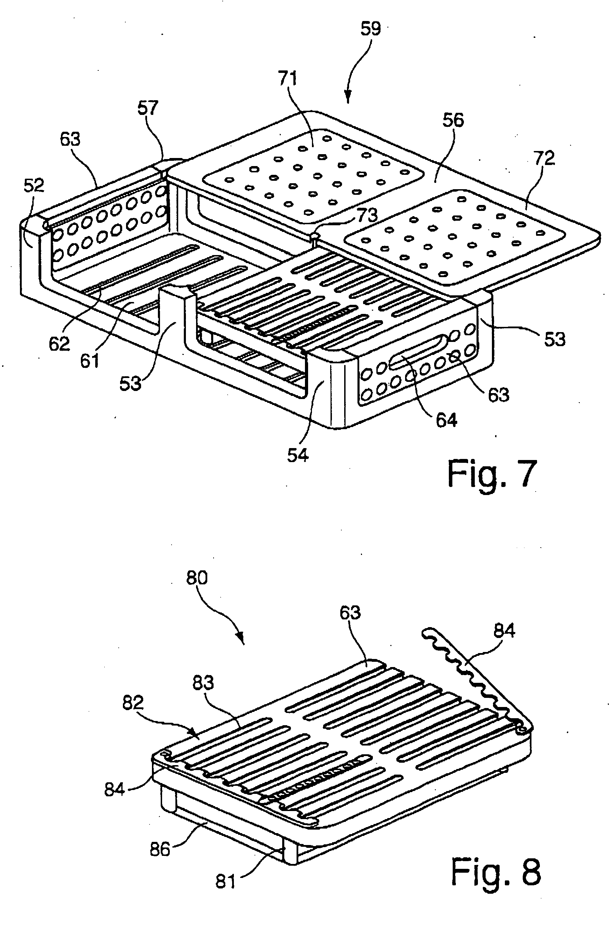

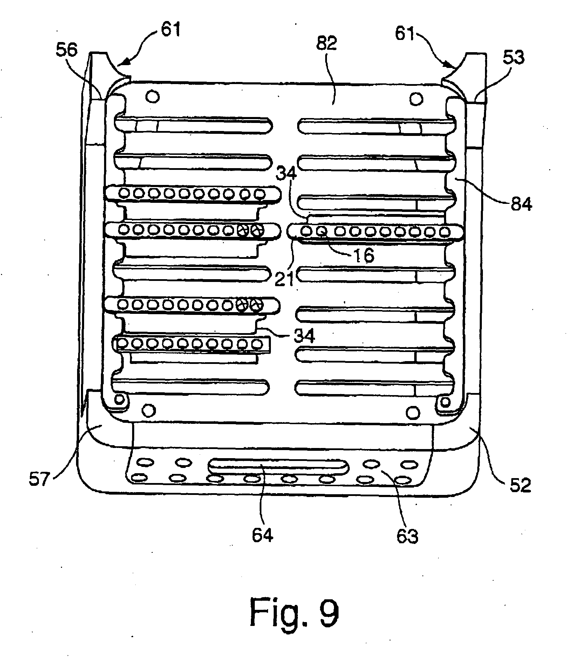

Das Lagerungssystem ist in den Fig. 5 bis 9 näher erläutert.

Der Grundkörper 50 des Lagerungssystems weist eine Bodenplatte 51 auf, die eine Vielzahl von Öffnungen oder Rillen 62 zum Durchtritt der Reinigungs- und Sterilisierflüssigkeit/Dampf umfasst. Die Öffnungen und Rillen 62 sollten dabei nicht so groß sein, dass man von unten in das Lagerungssystem hineingreifen kann. Ansonsten sind auch größere Öffnungen oder Rillen 62 im Boden 51 in Bezug auf eine gute Reinig/Sterilisierbarkeit durchaus erwünscht.

Die Bodenplatte 51 ist vorzugsweise aus Aluminium. Aluminium ist einerseits gegen die aggressiven, meist alkalischen Reinigungsflüssigkeiten hinreichend stabil, relativ leicht und ist zudem wärmeleitfähig, d.h. die Bodenplatte erwärmt sich während der Reinigung und Sterilisation hinreichend schnell und gibt auch die Wärme an die Halteabschnitte 53 ab.

Senkrecht zur Bodenplatte 51 sind vier, Hauptabschnitte 52, 54, 55, 57 angeordnet, die mit der Bodenplatte 51 starr verbunden sind. Diese Hauptabschnitte 52, 54, 56, 57 dienen als Führung für die auf der Bodenplatte zwischen den Hauptabschnitten 52, 54, 56, 57 anzuordnenden Einsätze, Tabletts oder Tische. Auch dienen die Hauptabschnitte 52, 54, 56, 57 zur Stabilisierung und - falls gewünscht - zur Aufnahme von einführbaren Seiten- oder Kopfseitenteilen 63. Vorzugsweise ist an jeder Ecke der Bodenplatte 51 jeweils ein Hauptabschnitt 52, 54, 55, 57 angeordnet und in Längsrichtung L befindet sich zwischen den beiden Hauptabschnitten 52, 54; 55, 57 jeweils noch ein weiterer Nebenabschnitt 53, 56, der zur Stabilisierung und Unterteilung des Grundkörpers 50 in zwei Bereiche 65, 66 dient. Hierfür weist jeder der beiden Nebenabschnitte 53, 56 jeweils zwei Führungsflächen 61 auf. Die Seitenflächen 58, 58' zwischen zwei Hauptabschnitten 52, 54 bzw. 55, 57 ist somit bis auf die Fläche des stegförmigen Hauptabschnittes 52, 54 bzw. 55, 57 und ggf. die Fläche des stegförmigen Nebenabschnittes 53 und der Fläche der Schiene 79 offen. Die Haupt- und Nebenabschnitte 52, 57 weisen verglichen mit der Gesamtlänge L eine geringe Breite auf.

Die Haupt- und Nebenabschnitte 52 bis 57 können auch untereinander über eine schmale Schiene 79 miteinander verbunden und die Schiene 79 an dem Boden 51 befestigt sein.

Insgesamt bilden die Haupt- und Nebenabschnitte, gegebenenfalls mit der Schiene 79 oder direkt mit dem Boden, eine Rahmen- oder Tragbalkenstruktur, insbesondere aus einem oder mehreren miteinander verbundenen U-förmigen Tragstrukturen (52, 79, 53; 53, 51, 54).

Die Haupt- und Nebenabschnitte 52, 53, 54, 55 und 57 weisen alle dieselbe Höhe h und an den Hauptabschnitten 52, 54, 55 und 57 und der senkrecht der Längsachse verlaufenden, nach innen weisenden Fläche des Kopfseitenteils 63 eine Nut 68 zum Einschieben des Deckels 59 auf.

Nachdem der Deckel 59 in Fig. 5 in Richtung des Pfeils 70 über den Nebenabschnitt 56 in die Nut 68 eingeschoben wird, ist die Höhe des Nebenabschnitts 56 gegenüber der anderen Abschnitte 52 - 55 und 57 verkürzt.

An der Stirnfläche des Lagerungssystems kann jeweils zwischen zwei Hauptabschnitten 52, 57 bzw. 54, 55 ein Kopfseitenteil 63 befestigt oder eingeschoben werden. Solche Kopfseitenteile 63 können herstellerseitig in verschiedenen Farben bereitgestellt werden, um dem Händler auf der Grundlage eines Grundkörpers 50 und verschiedener Kopfseitenteile 63 zu ermöglichen, für den Kunden das entsprechend einem bestimmten Schraubendurchmesser markierte Lagerungssystem auf einfache Weise bereit zu stellen. Der Händler kann das jeweils gewünschte Kopfseitenteil 63 in den Basiskörper 50 einschieben und dann durch Verschrauben, beispielsweise durch die Halteeinrichtungen, fixieren. Das Kopfseitenteil 63 weist ebenfalls Öffnungen 69 zur besseren Reinigung/Sterilisation und Griffmulden 64 auf.

Die Farbe des herstellerseitig einfügbaren Kopfseitenteils 63 korrespondiert mit der Farbe der Schraubenverpackung. Durch eine durchgängige farbige Markierung des Lagerungssystems und der Schraubenverpackungen kann sich der Arzt bei der Auswahl passender Schraubendurchmesser/Knochenplatten/Instrumente an der jeweiligen Farbe orientieren.

Die Kopfseitenteile 63 und die Abschnitte 52 bis 57 können aus Kunststoff oder Metall sein. Vorzugsweise sind sie aus PPSU.

Der Deckel 59 weist einen Rahmen 72 aus Metall auf und ist ansonsten im Wesentlichen transparent und aus Kunststoff. Öffnungen 71 im Deckel 59 ermöglichen einen Durchtritt der Reinigungs-/Sterilisierflüssigkeit. Durch die Transparenz des Deckels ist auch von oben die Einsicht in das Lagerungssystem möglich und beispielsweise durch die farbige Markierung überprüfbar, ob in dem Lagerungssystem die Schrauben passender Größe in der Schraubverpackung eingeschoben sind. Weiterhin weist der Deckel 59 eine Öffnung 73 auf, in die ein korrespondierender in dem Nebenabschnitt 53 angeordneter Stift 74 eingerastet werden kann.

Zur Aufnahme der zuvor beschriebenen Schraubenverpackungen ist ein zusätzlicher tischartiger Einsatz 80 vorgesehen, der von oben entlang der Führungsflächen 60, 61 der Haupt- und Nebenabschnitte 52, 53, 56, 57 oder 53, 54, 55, 56 auf den Boden 51 aufgestellt werden kann. Der tischartige Einsatz 80 weist vier Füße 81 und eine horizontale Platte 82 auf, in der mehrere längliche Öffnungen 83 vorgesehen sind, die zum seitlichen Einschieben der zuvor beschriebenen Schraubverpackungen 11 dienen. Zur Stabilisierung können die Füße miteinander an deren unterem Ende durch einen umfassenden Rahmen 86 verbunden sein. Im eingeschobenen Zustand liegen die Unterseite des Stegabschnitts 34 auf der horizontalen Platte 82 auf und die Randbereiche der horizontalen Platte 82 greifen in die Nut zwischen den Führungsmitteln 25, 24 und 28, 29 in der Halteeinrichtung 12 ein.

Auf den die Öffnungen 83 umfassenden Seiten ist an jeder Seite zusätzlich ein Schwenkarm 84 vorgesehen, um die in die länglichen Öffnungen 83 eingeschobenen Schraubverpackungen zu fixieren und ein Hängenbleiben oder die Gefahr von Verletzungen an den nach außen weisenden Öffnungen 83 zu verhindern.

Aufgrund der Zugänglichkeit des Schwenkarms 84 infolge der im Vergleich zum Stand der Technik fehlenden Seitenwand ist ein Öffnen und Schließen des Schwenkarms 84 und damit ein Einschieben und Entfernen der Schraubenverpackungen 11 und auch deren Schutzabdeckung 13 oder Deckel 22 in dem in dem Lagerungssystem befindlichen tischartigen Einsatz 80 möglich.

Nachdem die Öffnungen 83 von beiden Seiten des tischartigen Einsatzes 80 zugänglich sind, kann das Lagerungssystem von beiden Längsseiten be- und entladen werden.

Sind die Schutzabdeckung 13 und der Deckel 22 bei einer in dem tischartigen Einsatz 80 und in dem Lagerungssystem befindlichen Schraubenverpackungen 11 entfernt, so ist ein ungehinderter Durchtritt der Reinigungsflüssigkeit von der Seite an die frei in der Halteeinrichtung 13 hängenden Schraubenschäfte möglich.

## Patentansprüche

1. Vorrichtung zum Verpacken, Aufbewahren und/oder Lagern von Implantat-Schrauben, -Stiften und -Klammern, wobei
die Vorrichtung (11) dreiteilig aus einer Halteeinrichtung (12), einer Schutzabdeckung (13) und einem Deckel (22) ausgebildet ist,
die Halteeinrichtung (12) wenigstens eine Aufnahme (16) für Schrauben (14), Stifte oder Klammern aufweist,
die Schutzabdeckung (13) und der Deckel (22) an der Halteeinrichtung (12) anbringbar sind und
die Schutzabdeckung (13) die aus der Halteeinrichtung (12) nach unten herausragenden Abschnitte (15) der durch die Halteeinrichtung (12) aufgenommenen Schrauben (14), Stifte oder Klammern umgibt, **dadurch gekennzeichnet, dass**
die Halteeinrichtung (12) wenigstens einen an einem horizontalen Wandabschnitt (19) oder einem vertikalen Wandabschnitt (20) angeordneten Stegabschnitt (34) aufweist, der parallel zum horizontalen Wandabschnitt angeordnet ist und als Informationsfläche dienen kann.

2. Vorrichtung nach Anspruch 1, **dadurch gekennzeichnet, dass** die Schutzabdeckung (13) eine Öffnung (17) zur Befestigung an der Halteeinrichtung (12) aufweist.

3. Vorrichtung nach Anspruch 2, **dadurch gekennzeichnet, dass** die Schutzabdeckung (13) ein Gehäuse mit einer Öffnung (17) ist, wobei zusätzlich ein rechtwinklig zur Verschiebe-/Montagerichtung (37) verlaufender Wandabschnitt (18) freigeschnitten ist.

4. Vorrichtung nach einem der Ansprüche 1 bis 3, **dadurch gekennzeichnet, dass** die Aufnahmen (16) in der Halteeinrichtung (12) Bohrungen (16) in dem horizontalen Wandabschnitt (19) der Halteeinrichtung (12) sind.

5. Vorrichtung nach einem der Ansprüche 1 bis 4, **dadurch gekennzeichnet, dass** mehrere Aufnahmen (16) oder Bohrungen (16) vorgesehen sind, die entlang einer Geraden angeordnet sind.

6. Vorrichtung nach einem der Ansprüche 4 oder 5, **dadurch gekennzeichnet, dass** senkrecht zum horizontal verlaufenden Wandabschnitt (19) der vertikal verlaufender Wandabschnitt (20) vorgesehen ist, der mit der nach außen weisenden Kante des horizontalen Wandabschnitts (19) starr verbunden ist.

7. Vorrichtung nach einem der Ansprüche 1 bis 6, **dadurch gekennzeichnet, dass** der Deckel (22) die aus der Halteeinrichtung (12) nach oben herausragenden Köpfe (23) der durch die Halteeinrichtung (12) aufgenommenen Schrauben (14), Stifte oder Klammern umgibt.

8. Vorrichtung nach Anspruch 6 oder 7, **dadurch gekennzeichnet, dass** an dem vertikalen Wandabschnitt (20) der Halteeinrichtung (12) Führungsmittel (24, 25, 26, 27, 28, 29) vorgesehen sind, die mit komplementären Führungsmitteln (30, 31) an der Schutzabdeckung (13) und/oder Führungsmitteln (32, 33) an dem Deckel (22) korrespondieren.

9. Vorrichtung nach einem der Ansprüche 1 bis 8, **dadurch gekennzeichnet, dass** wenigstens Teile der Vorrichtung farbkodiert sind.

10. Vorrichtung nach einem der Ansprüche 1 bis 9, **dadurch gekennzeichnet, dass** Messskalen zur Bestimmung der Schraubenlänge an der Vorrichtung vorgesehen sind.

## Claims

1. Device for packaging and storing implant screws, implant pins and implant clamps, which device (11) is made up of three parts, namely a holder (12), a protective cover (13) and a lid (22), of which the holder (12) has at least one receiver (16) for screws (14), pins or clamps, the protective cover (13) and the lid (22) can be fitted on the holder (12), and the protective cover (13) surrounds those sections (15) of the screws (14), pins or clamps received by the holder (12) that protrude downwards from the holder (12), **characterized in that** the holder (12) has at least one web section (34), which is arranged on a horizontal wall section (19) or a vertical wall section (20) and which is arranged parallel to the horizontal wall section and can serve as an information surface.

2. Device according to Claim 1, **characterized in that** the protective cover (13) has an opening (17) for attachment to the holder (12).

3. Device according to Claim 2, **characterized in that** the protective cover (13) is a housing with an opening (17), and a wall section (18) that extends at right angles to the displacement/assembly direction (37) is additionally cut free.

4. Device according to one of Claims 1 to 3, **characterized in that** the receivers (16) in the holder (12) are bores (16) in the horizontal wall section (19) of the holder (12).

5. Device according to one of Claims 1 to 4, **characterized in that** a plurality of receivers (16) or bores (16) are provided and are arranged along a straight line.

6. Device according to one of Claims 4 and 5, **characterized in that** the vertical wall section (20) is provided perpendicular to the horizontal wall section (19) and is rigidly connected to the outwardly facing edge of the horizontal wall section (19).

7. Device according to one of Claims 1 to 6, **characterized in that** the lid (22) surrounds the heads (23), protruding upwards from the holder (12), of the screws (14), pins or clamps received by the holder (12).

8. Device according to Claim 6 or 7, **characterized in that** the vertical wall section (20) of the holder (12) is provided with guide means (24, 25, 26, 27, 28, 29) which correspond with complementary guide means (30, 31) on the protective cover (13) and/or guide means (32, 33) on the lid (22).

9. Device according to one of Claims 1 to 8, **characterized in that** at least parts of the device are colour-coded.

10. Device according to one of Claims 1 to 9, **characterized in that** measurement scales for determining the screw length are provided on the device.

## Revendications

1. Dispositif d'emballage, de conservation et/ou d'entreposage de vis, de tiges et de pinces à implants,
le dispositif (11) étant configuré en trois parties, à savoir un dispositif de maintien (12), un recouvrement de protection (13) et un couvercle (22),
le dispositif de maintien (12) présentant au moins un logement (16) pour des vis (14), des tiges ou des pinces,
le recouvrement de protection (13) et le couvercle (22) pouvant être placés sur le dispositif de maintien (12) et
le recouvrement de protection (13) entourant les parties (15) des vis (14), des tiges ou des pinces reprises par le dispositif de maintien (12) qui débordent vers le bas par rapport au dispositif de maintien (12),
**caractérisé en ce que**
le dispositif de maintien (12) présente sur une partie horizontale de paroi (19) ou sur une partie verticale de paroi (20) au moins une partie en nervure (34) disposée parallèlement à la partie horizontale de paroi et pouvant servir de surface d'information.

2. Dispositif selon la revendication 1, **caractérisé en ce que** le recouvrement de protection (13) présente une ouverture (17) de fixation sur le dispositif de maintien (12).

3. Dispositif selon la revendication 2, **caractérisé en ce que** le recouvrement de protection (13) est un boîtier doté d'une ouverture (17) et **en ce qu'**une partie de paroi (18) qui s'étend perpendiculairement à la direction de coulissement et/ou de montage est en outre découpée.

4. Dispositif selon l'une des revendications 1 à 3, **caractérisé en ce que** les logements (16) prévus dans le dispositif de maintien (12) sont des alésages (16) ménagés dans la partie horizontale de paroi (19) du dispositif de maintien (12).

5. Dispositif selon l'une des revendications 1 à 4, **caractérisé en ce qu'**il présente plusieurs logements (16) ou alésages (16) disposés en ligne droite.

6. Dispositif selon l'une des revendications 4 ou 5, **caractérisé en ce que** la partie verticale de paroi (20) qui est reliée rigidement au bord tourné vers l'extérieur de la partie horizontale de paroi (19) est prévue perpendiculairement à la partie horizontale de paroi (19).

7. Dispositif selon l'une des revendications 1 à 6, **caractérisé en ce que** le couvercle (22) entoure les têtes (23), qui débordent vers le haut par rapport au dispositif de maintien (12), des vis (14), des tiges ou des pinces reprises dans le dispositif de maintien (12).

8. Dispositif selon les revendications 6 ou 7, **caractérisé en ce que** des moyens de guidage (24, 25, 26, 27, 28, 29) qui correspondent à des moyens complémentaires de guidage (30, 31) prévus sur le recouvrement de protection (13) et/ou à des moyens de guidage (32, 33) prévus sur le couvercle (22) sont prévus sur la partie verticale de paroi (20) du dispositif de maintien (12).

9. Dispositif selon l'une des revendications 1 à 8, **caractérisé en ce qu'**au moins certaines parties du dispositif sont codées en couleurs.

10. Dispositif selon l'une des revendications 1 à 9, **caractérisé en ce que** des échelles de mesure sont prévues pour déterminer la longueur des vis sur le dispositif.
